(19) Europäisches Patentamt
European Patent Office
Office européen des brevets

(11) **EP 2 688 999 B1**

(12) **EUROPEAN PATENT SPECIFICATION**

(45) Date of publication and mention
of the grant of the patent:
**24.02.2016 Bulletin 2016/08**

(21) Application number: **11745741.6**

(22) Date of filing: **01.08.2011**

(51) Int Cl.:
*C11D 3/37* (2006.01)    *C08F 220/28* (2006.01)
*C08L 33/14* (2006.01)    *A61Q 5/00* (2006.01)
*A61Q 5/02* (2006.01)     *A61Q 5/06* (2006.01)
*A61K 8/81* (2006.01)     *A61Q 19/02* (2006.01)
*A61Q 19/10* (2006.01)    *C08F 220/06* (2006.01)
*C08F 220/18* (2006.01)   *C08F 222/28* (2006.01)

(86) International application number:
**PCT/EP2011/063187**

(87) International publication number:
**WO 2011/117427 (29.09.2011 Gazette 2011/39)**

(54) **DETERGENT COMPOSITIONS**

WASCHMITTELZUSAMMENSETZUNGEN

COMPOSITIONS DÉTERGENTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.03.2011 IT VA20110008**

(43) Date of publication of application:
**29.01.2014 Bulletin 2014/05**

(73) Proprietor: **Lamberti SpA
21041 Albizzate (IT)**

(72) Inventors:
• **COGET, Karine
I - 21019 Somma (VA) (IT)**
• **FUMAGALLI, Chiara
I-21010 Besnate (VA) (IT)**

• **BALDARO, Eva
I-20127 Milano (IT)**
• **BENETTI, Arianna
I-21018 Sesto Calende (VA) (IT)**
• **FLORIDI, Giovanni
I-28100 Novara (IT)**
• **LI BASSI, Giuseppe
I-21026 Gavirate (VA) (IT)**

(74) Representative: **Giaroni, Paola
LAMBERTI S.p.A.
Ufficio Brevetti
Via Piave, 18
21041 Albizzate (VA) (IT)**

(56) References cited:
**US-A- 4 529 773     US-A1- 2005 131 176**

## Description

### Technical field

The present invention relates to liquid aqueous detergent compositions comprising as thickeners and suspending agents crosslinked alkali swellable polyacrylates containing one or more acetoacetyl or cyanoacetyl groups. The crosslinked alkali swellable polyacrylates containing one or more acetoacetyl or cyanoacetyl groups possess high thickening capability in the presence of surfactants and electrolytes, provide homogeneous and clear solutions and give excellent performances in terms of suspending properties.

### Background art

It is known that a technical problem often encountered in the detergent & toiletries industries is to obtain stable viscous homogeneous formulations comprising surfactants, electrolytes and possibly dispersed solid particles. The thickeners employed shall develop their thickening capability without negatively altering the other properties of the formulations.

Most often, thickeners are also asked to control the whole rheology of the compositions, including the yield value.

In the field of liquid detergent aqueous compositions, the thickeners are generally selected among crosslinked polyacrylic acids and crosslinked acrylic acid copolymers (belonging to the class of crosslinked alkali swellable polyacrylates), guar gum and its derivatives, starch and its derivatives, crosslinked polyoxyethylene, carboxymethylcellulose, partially hydrolyzed crosslinked water swellable polymers such as partially hydrolyzed polyacrylamides, associative polyurethanes.

Crosslinked polyacrylic acids and crosslinked acrylic acid copolymers partially neutralized to the sodium salts are often preferred.

In the specialised literature many methods are reported to regulate the rheological properties of different formulations including the use of crosslinked acrylic acid copolymers performing as thickeners at neutral and basic pH.

We cite as an example:

- GB 870,994, disclosing copolymers of methacrylic acid and $C_1$-$C_4$ alkyl acrylate in aqueous emulsion;
- EP 13836, describing thickeners based on copolymers of (meth)acrylic acid, $C_1$-$C_4$ (meth)acrylic esters, selected (meth)acrylic esters comprising polyoxyethylene units, and optionally polyethylenically unsaturated monomers; the thickeners are said to be less sensitive to electrolytes than prior art synthetic thickeners that are prepared without the use of the selected (meth)acrylic esters;
- US 4,529,773 and US 4,138,381, describing alkali-soluble emulsion acrylic polymers comprising associative monomers and a method for their use;
- US 6,140,438, describing crosslinked acrylic polymers possessing high thickening and suspending properties obtainable by polymerization of acrylic monomers in the presence of specific crosslinking agents;
- EP 1272159, describing the use of crosslinked acrylate copolymers generally free from, i.e. containing less than about 1% by weight, associative monomers.

### Summary of the disclosure

In accordance with the present disclosure liquid aqueous detergent compositions are prepared comprising:

i) from 0.2 to 10 % by weight, preferably from 0.5 to 8 % by weight, of a thickening agent which is a crosslinked alkali swellable polyacrylate obtainable by polymerization of:

a) from 20 to 70% by weight, preferably from 20 to 50 % by weight, of a monoethylenically unsaturated monomer containing a carboxylic group;
b) from 20 to 70% by weight, preferably from 40 to 70 % by weight, of a (meth)acrylic acid ester;
c) from 0.05 to 3% by weight, preferably from 0.1 to 2 % by weight, of an unsaturated monomer containing one or more acetoacetyl or cyanoacetyl groups;
d) from 0.01 to 3% by weight, preferably from 0.02 to 1 % by weight, of a polyethylenically unsaturated monomer;
e) from 0 to 10% by weight of a nonionic acrylic associative monomer;

ii) from 5 to 60% by weight, preferably from 5 to 20 % by weight, of a detergent component consisting of at least one compound selected from anionic surfactants, amphoteric surfactants, cationic surfactants, zwitterionic surfactants, non-ionic surfactants and mixture thereof.

The above described crosslinked alkali swellable polyacrylates have proved to efficiently perform as thickeners in the presence of electrolytes and surfactants and possess improved suspending and thickening properties in comparison with crosslinked alkali swellable polyacrylates of the prior art.

It is therefore a further object of the present disclosure the use of the above described crosslinked alkali swellable polyacrylates for thickening liquid aqueous detergent compositions comprising from 5 to 60% by weight, preferably from 5 to 20% by weight, of the above detergent component.

**Detailed description of the disclosure**

With the expression "detergent compositions" we mean the products normally used both for personal detergence (personal care detergents, such as shampoos and skin and body cleansers) and for household or heavy duty detergence (household and heavy duty detergents, such as laundry detergents, dishwashing detergents household cleansers, light duty detergents and industrial detergents).

A key point in the preparation of the thickener of the present invention is the presence among the monomers of an unsaturated monomer containing one or more acetoacetyl or cyanoacetyl groups.

The acetoacetyl and cyanoacetyl groups respectively have the formula $-CO-CH_2-COCH_3$ and $-CO-CH_2-CN$.

Examples of useful unsaturated monomers containing one or more acetoacetyl or cyanoacetyl groups are acetoacetoxyalkyl (meth)acrylates, allyl acetoacetates, vinyl acetoacetates, unsaturated acetoacetamides, allylcyanoacetates.

Preferably the unsaturated monomer containing one or more acetoacetyl or cyanoacetyl groups is an unsaturated monomer containing one or more acetoacetyl group.

More preferably the unsaturated monomer containing one or more acetoacetyl groups is an acetoacetoxyalkyl (meth)acrylate, and in particular an acetoacetoxyalkyl (meth)acrylate of formula $CH_2=CR-CO-O-Ri-O-CO-CH_2-CO-CH_3$, in which R is H or $CH_3$ and $R_1$ is a $C_2-C_4$ linear or branched alkylene radical group.

Most preferably the unsaturated monomer containing one or more acetoacetyl groups is acetoacetoxyethyl methacrylate, i.e. an acetoacetoxyalkyl (meth)acrylate) of formula $CH_2=CR-CO-O-R_1-O-CO-CH_2-CO-CH_3$ in which R is $CH_3$ and $R_1$ is the ethylene radical.

Acetoacetoxyethyl methacrylate has a reactive methylene group that is useful for crosslinking and modifying the polymer after its synthesis.

The reactive methylene group is known to react with melamines, isocyanates, Michael reactive double bonds, aldehydes.

Beside the reactive methylene group, acetoacetoxyalkyl (meth)acrylates contain a reactive carbonyl group, which also undergoes reactions that are useful for crosslinking or modification of resins.

The carbonyl group of acetoacetylated polymers is in equilibrium between its enol and keto forms: enolization enables chelation of the acetoacetylated polymer with polyvalent cations from metals, such as zinc, tin, aluminum, copper, zirconium, and reaction with amines and diamines.

Because of this peculiar chemistry, acetoacetoxyethyl methacrylate finds use as an acrylic monomer for preparing post reaction crosslinkable polymers for coatings, plastics and adhesive applications.

By way of example, US 5,516,453, describes a stable ambient curing one-pack composition containing an active methylene-functional component and an active methylene-reactive component that may be used as coating, adhesive and impregnating agent and that can be applied on a variety of substrates, such as wood, cement and concrete, fabrics, metals, ceramics, plastics.

Beside the unsaturated monomer containing reactive methylene groups, the crosslinked alkali swellable polyacrylates of the disclosure are obtained by co-polymerization of one or more monoethylenically unsaturated monomers containing a carboxylic group, one or more (meth)acrylic esters, one or more polyethylenically unsaturated monomer and, possibly, one or more nonionic acrylic associative monomer.

Acrylic acid, methacrylic acid, itaconic acid and mixtures thereof are examples of monoethylenically unsaturated monomer containing a carboxylic group that are useful for the preparation of the crosslinked alkali swellable polyacrylates of the present disclosure.

Methacrylic acid is the preferred monoethylenically unsaturated monomer containing a carboxylic group.

Useful (meth)acrylic acid esters are $C_1C_{40}$ (meth)acrylic acid alkyl ester, such as methyl, ethyl, propyl, butyl, 2-ethylhexyl, lauryl (meth)acrylates and mixtures thereof.

Preferably the (meth)acrylic acid ester is ethyl acrylate.

The addition of the polyethylenically unsaturated monomer, acting as polymer crosslinker, is a further key point in the preparation of the thickeners of the present invention.

The polyethylenically unsaturated monomer can be any of the known polyfunctional derivatives that are known to undergo radical polymerization with (meth)acrylic monomers.

Among the useful polyethylenically unsaturated monomer we cite diallyl maleate, allyl methacrylate, diallyl phthalate, N-methylene-bis-acrylamide, pentaerithritol ether polyacrylates, triallyl cianurate.

The nonionic acrylic associative monomer may be selected among (meth)acrylic acid esters of $C_8$-$C_{30}$ alkyl, alkylaryl or polycyclic hydrocarbyl monoether of a polyethylene glycol having at least two oxyethylene units, preferably having 10 to 40 oxyethylene units, and having up to 70 oxyethylene units, this ester having general formula $H_2C=C(R)$-CO-$O(CH_2CH_2O)_n$-R', wherein

R is H or $CH_3$, the latter being preferred,

n is at least 2, and preferably has an average value of at least 10, up to 40 to 60 or even up to 70 or so, and R' is a hydrophobic group, for example an alkyl, alkylaryl, or polycyclic alkyl group having 8 to 30 carbon atoms, preferably 16 to 18 carbon atoms, or more preferably having an average of 12 to 18 carbon atoms.

Other unsaturated monomers may be used in the polymerization beside the monomers a) to e), such as, by way of example, other nonionic acrylic monomers, monoethylenically unsaturated monomers, possibly containing a sulfonic acid group, cationic acrylic monomers.

Non limiting examples of other utilizable unsaturated monomers are vinyl acetate, styrene, vinyl chloride, vinylidene chloride, acrylonitrile, (meth)acrylamide, N,N,-dimethyl(meth)acrylamide, t-butyl-(meth)acrylamide, sodium vinyl sulfonate, 2-acrylamido-2-methylpropane sulfonic acid.

Nonetheless, by using only the monomers from a) to e) in polymerization, excellent thickening and suspending performances of the crosslinked alkali swellable polyacrylates in aqueous detergent compositions are obtained. The crosslinked alkali swellable polyacrylates of the disclosure can also indirectly be obtained by reaction of the monomers a), b), d) and possibly e), in the presence of an unsaturated monomer comprising a hydroxyl group and post synthesis reaction of the obtained polymer with commercially available t-butyl acetoacetate.

The crosslinked alkali swellable polyacrylate has high Brookfield® viscosity at pH 7.5 in water, i.e. has Brookfield® viscosity (spindle 6, RVT, 20 rpm, 1.0% by weight and 20°C) of at least 500 mPa∗s.

The detergent component of the liquid aqueous detergent composition of the invention is made of anionic surfactants, amphoteric surfactants, cationic surfactants, non-ionic surfactants, zwitterionic surfactants and mixtures thereof.

Preferably, the detergent component of the liquid aqueous detergent composition of the invention comprises anionic surfactants.

According to an embodiment of the disclosure, the detergent component of the liquid aqueous detergent composition of the invention consists of anionic surfactants.

Anionic surfactants include alkyl and alkyl ether sulfates;alkyl sulfonates; alkyl and alkyl ether phosphates; alkyl or alkyl ether sulfosuccinates and alkyl and alkyl ether carboxylates or anionic derivatives of alkyl polyglycosides, such as the citric, tartaric or sulfosuccinic ester of alkyl polyglucosides.

The amphoteric surfactants which can be used in the composition of the present disclosure are those which can be broadly described as derivatives of aliphatic quaternary ammonium compounds, wherein one of the aliphatic substituents contains an anionic water-solubilizing group, e.g., carboxylate, sulfonate, sulfate etc. Examples of amphoteric surfactants include cocoamphocarboxypropionate, cocoamphocarboxy propionic acid, cocoamphoacetate, cocoamphodiacetate, sodium lauroamphoacetate.

Cationic surfactants useful in the compositions of the present disclosure contain amino or quaternary ammonium hydrophilic moieties which are positively charged when dissolved in an aqueous composition of the present invention. Examples of cationic surfactants are long-chain alkyl trimethyl ammonium chloride, long-chain alkyl benzyl dimethyl ammonium chloride, alkylamine hydrochlorides, alkylamine acetates and di(long-chain alkyl) dimethyl ammonium bromide.

Nonionic surfactants can be broadly defined as compounds containing a hydrophobic moiety and a nonionic hydrophilic moiety. Examples of the hydrophobic moiety can be alkyl, alkyl aromatic, and aryl aromatic. Examples of hydrophilic moieties are polyoxyalkylenes, amine oxides, and alkanol amides. Examples of non ionic surfactants are alkoxylated fatty alcohols or fatty acids, alkoxylated di- and tri-stiryl phenols, polyhydroxy fatty acid amides, sugar esters and polyesters, alkoxylated sugar esters, sorbitan and alkoxylated sorbitan fatty acid esters. Other examples of nonionic emulsifiers include alkyl polyglycosides, such as coco polyglucosides.

Examples of zwitterionic surfactants include alkyl betaines and amido betaines, sultaines, alkyl glycinates and alkyl carboxyglycinates. Surprisingly, the crosslinked alkali swellable polyacrylates of the disclosure containing reactive methylene groups show an enhanced Brookfield® viscosity in water in the presence of surfactants. A wide range of surfactant type and amount is effective.

Although it is known from the prior art that the introduction of specific associative monomers in crosslinked alkali swellable polyacrylates may enhance the thickening performance in the presence of surfactants, the remarkable enhancement of thickening that has been observed upon the addition of a surfactant to an aqueous system containing the thickeners of the invention is independent from the presence of nonionic acrylic associative monomers in the polymer.

This phenomenon is not observed in the same way with analogous crosslinked alkali swellable polyacrylates of the prior art that do not contain acetoacetyl or cyanoacetyl groups.

What more, it has been found that even little amounts of monomer containing reactive methylene groups, significantly improve the suspending properties of aqueous detergent compositions.

According to one of the preferred embodiments of the disclosure, to enhance the suspending properties of the thickeners, from 0.1 to 0.5 wt % of the unsaturated monomer containing one or more acetoacetyl or cyanoacetyl groups is used in polymerization, together with from 1.0 to 2.0 wt% of the nonionic acrylic associative monomer.

Advantageously, the reactive methylene group of the thickeners of the aqueous liquid detergent compositions of the disclosure may further provide tunable viscosifying properties to the polymer in the presence of several common additives of detergent compositions, such as preservatives (in particular formaldehyde or formaldehyde donors) and polyvalent metal cations.

The amount and kind of both the reactive methylene groups in the polymer and the additives may serve to tune the viscosity of the final liquid aqueous compositions, as the person skilled in the art would easily determine with few experiments.

The crosslinked alkali swellable polyacrylates of the present disclosure may be prepared in any conventional manner, such as for instance, by precipitation polymerization, suspension and solution polymerization, emulsion polymerization. Generally, the crosslinked alkali swellable polyacrylates of the present disclosure are prepared by emulsion polymerization.

The emulsion polymerization techniques are well known in the art such as, for example, as disclosed in U.S. Patents No. 4,325,856 ; 4,654,397 ; and 4,814,373 . Conventional surfactants may be used in emulsion polymerization, such as anionic and/or nonionic emulsifiers, for example, alkali metal or ammonium alkyl sulfates, alkyl sulfonic acids, fatty acids, and oxyethylated alkyl phenols. The amount of surfactant used is usually 0.1 % to 6% by weight, based on the weight of total monomer. Thermal or redox initiation processes may be used. Conventional free radical initiators may be used such as, for example, hydrogen peroxide, t-butyl hydroperoxide, t-amyl hydroperoxide, alkali or ammonium persulfates, and azo initiators such as 4,4'-azobis(4-cyanopentanoic acid), and 2,2'-azobisisobutyronitrile ("AIBN"), typically at a level of 0.01 % to 3.0% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymers. The monomer mixture may be added neat or as an emulsion in water. The monomer mixture may be added in a single addition or in multiple additions or continuously over the reaction period using a uniform or varying composition. The emulsion polymerization process may utilize a preformed seed emulsion polymer such as, for example, by adding 5% (based on total monomer) of the monomer mixture to the kettle and making it react previously. Techniques to reduce residual monomer such as, for example, subjecting the reaction mixture to steam stripping, hold times, and additional radical sources may be employed.

The crosslinked alkali swellable polyacrylates of the present disclosure are generally supplied in their acidic form, in emulsion or in solid form; as they contain acidic groups, they need to be neutralized to the salt form to develop optimal viscosity increase in the aqueous detergent compositions. Typically, laundry detergents and many household cleansers have neutral to basic pH values; as a consequence, the aqueous detergent compositions of the disclosure may generally be prepared by simple dilution of the acidic emulsion of the crosslinked alkali swellable polyacrylates or by dissolution of the crosslinked alkali swellable polyacrylates in solid form in a nearly neutral or basic aqueous solution, accompanied by the addition of the detergent component and of any optional ingredient.

Surfactants, which are essential ingredients of the aqueous detergent compositions, can be added after or before the crosslinked alkali swellable polyacrylates, but are preferably added after the crosslinked alkali swellable polyacrylates; if needed, for the neutralization of the crosslinked alkali swellable polyacrylates, high diffusion alkalis are commonly used, such as sodium or potassium hydroxyde, ethanolamine, ammonia, etc.. Subsequently, the pH of the obtained thickened composition may also be lowered, when and how advisable, without significant impairment of the viscosity level; typically weak organic acids, such as citric acid, salicylic acid and the like may be used.

Additional ingredients of the liquid aqueous detergent compositions of the disclosure are those commonly known in the art and, by way of example, may be selected among perfumes, dyes, pearlescent agents, opacifiers, enzymes, preservatives, disinfecting agents, anti-redeposition aids, zeolite builders, phosphate builders, antimicrobial additives, foaming agents, antifoam agents, humectants, conditioning agents, soil release agents, brighteners, solvents and pH buffering means.

**Examples 1-6**

The thickened liquid aqueous detergent compositions of the Examples 1-6 were prepared according to the following procedure.

The crosslinked alkali swellable polyacrylates emulsion is prepared by emulsion polymerization and dispersed in deionized water at 2.7 wt% a.m.. Then, the detergent component is added (10.7 wt % a.m.) and after mixing the pH is adjusted to 6.6-6.8 by addition of NaOH. The solution is further mixed by stirring for about 30 minutes, and allowed to stand overnight before the viscosity is measured.

Table 1a describes the detergent component names and chemical compositions used in the Examples 1 to 6, while Table 1b describes the monomer composition of the two crosslinked alkali swellable polyacrylates tested (PAC1 and PAC2); the quantities of monomers are in parts by weight (pbw).

The detergent components of Example 1 and 4 to 6 are commercialized by Zschimmer & Schwarz, while the detergent components of Examples 2 and 3 are commercialized by Lamberti SpA.

**Table 1a - Detergent component**

| Examples | Trade name | Chemical description |
|---|---|---|
| Example 1 | ZETESOL LES 2/SL | Laureth (2EO) sulphate sodium salt 27 wt% a.m. (unpreserved) |
| Example 2 | ROLPON 24/230[*] | Laureth (2EO) sulphate sodium salt 27 wt% a.m. |
| Example 3 | ROLPON LS | Sodium lauryl sulphate 30 wt% a.m. |
| Example 4 | SULFETAL LA | Ammonium lauryl sulphate 27 wt% a.m. |
| Example 5 | ZETESOL LA-370 | Ammonium laureth(3EO) sulphate salt, 70 wt% a.m. |
| Example 6 | SETACIN 103 Spezial | Disodium laureth sulfosuccinate, 40 wt% a.m. |

**Table 1b - Polyacrylates**

| Monomer | PAC1* | PAC2 |
|---|---|---|
| Ethyl acrylate (pbw) | 62 | 62 |
| Methacrylic acid (pbw) | 38 | 38 |
| Acetoacetoxyethyl methacrylate (pbw) | - | 1.00 |
| Diallylmaleate (pbw) | 0.05 | 0.05 |
| *comparative | | |

The viscosities of the thus obtained thickened liquid aqueous detergent compositions were measured using a Brookfield(R) Model LVT Viscometer at 25°C and at the indicated spindle speed, and are reported in Table 2 and Table 3 (BV).

A measure of the pseudoplasticity of the compositions is the Brookfield Yield Value (BYV), which is calculated from the following formula :

$$BYV = (BV_{0.5\ rpm} - BV_{1\ rpm})/100$$

The BYVs are also reported in Table 2 and Table 3.

**Table 2 - BV (mPa*s) and BYV**

| | Example 1 | | Example 2 | | Example 3 | |
|---|---|---|---|---|---|---|
| | BV | | | | | |
| rpm | PAC1* | PAC2 | PAC1* | PAC2 | PAC1* | PAC2 |
| 0.5 | 14480 | 29000 | 68000 | 380000 | 19800 | 568000 |
| 1 | 8840 | 18000 | 46400 | 254000 | 11000 | 300000 |

(continued)

|  | Example 1 | | Example 2 | | Example 3 | |
|---|---|---|---|---|---|---|
|  | BV | | | | | |
| rpm | PAC1* | PAC2 | PAC1* | PAC2 | PAC1* | PAC2 |
| 5 | 3248 | 5400 | 16280 | 197000 | 4000 | 136000 |
| 10 | 2172 | 3470 | 10860 | 133000 | 2750 | 90000 |
| 20 | 1510 | 2245 | 7430 | 80000 | 1850 | 52000 |
| 50 | 1042 | 1350 | 1044 | 40000 | 1150 | 25400 |
| 100 | 758 | 950 | 757 | 23000 | 785 | 14720 |
|  | BYV | | | | | |
|  | 56 | 110 | 220 | 1260 | 88 | 2680 |
| * comparative | | | | | | |

**Table 3 - BV (mPa*s) and BYV**

|  | Example 4 | | Example 5 | | Example 6 | |
|---|---|---|---|---|---|---|
|  | BV | | | | | |
| rpm | PAC1* | PAC2 | PAC1* | PAC2 | PAC1* | PAC2 |
| 0.5 | 21000 | 350000 | 33000 | 40000 | 36000 | 320000 |
| 1 | 13600 | 300000 | 22000 | 25000 | 22000 | 220000 |
| 5 | 4500 | 180000 | 6700 | 7400 | 6700 | 78000 |
| 10 | 2850 | 132000 | 4280 | 4670 | 4160 | 53000 |
| 20 | 1940 | 85000 | 2750 | 3000 | 2700 | 31000 |
| 50 | 1171 | 42000 | 1590 | 1728 | 1510 | 15000 |
| 100 | 830 | 24000 | 1130 | 1240 | 1086 | 8500 |
|  | BYV | | | | | |
|  | 74 | 500 | 110 | 150 | 140 | 1000 |
| *comparative | | | | | | |

### Example 7

The thickened liquid aqueous detergent compositions of Example 7 were prepared according to the following procedure.

The crosslinked alkali swellable polyacrylates were prepared by emulsion polymerization and dispersed in deionized water at 2.7 wt% a.m. and at 2.25 wt% a.m.. The detergent component is added (10.7 wt% a.m. of Sodium Laureth Sulfate and 2.4 wt% a.m. of Cocoamidopropyl Betaine) and mixed, then the pH is adjusted to 6.6-6.8 by addition of NaOH. The solutions are mixed by stirring for about 30 minutes and allowed to stand overnight before the viscosity is measured.

Four crosslinked alkali swellable polyacrylates having the monomer compositions reported in Table 4 (PAC3, PAC4, PAC5, PAC6) were tested; ; the quantities of monomers are in parts by weight (pbw).

**Table 4 - Polyacrylates**

| Monomer | PAC3 | PAC4 | PAC5 | PAC6* |
|---|---|---|---|---|
| Ethyl acrylate (pbw) | 62 | 62 | 62 | 62 |

(continued)

| Monomer | PAC3 | PAC4 | PAC5 | PAC6* |
|---|---|---|---|---|
| Methacrylic acid (pbw) | 38 | 38 | 38 | 38 |
| polyethoxylated $C_{16}$-$C_{18}$ alcohols methacrylates (20EO) (pbw) | 1.7 | 1.7 | 1.7 | 1.7 |
| Acetoacetoxyethyl methacrylate (pbw) | 0.1 | 0.2 | 0.3 | - |
| Diallylmaleate (pbw) | 0.05 | 0.05 | 0.05 | 0.05 |
| *comparative | | | | |

The viscosities (BV, mPa$_*$s) and BYVs of the thus obtained thickened liquid aqueous detergent compositions were measured using a Brookfield(R) Model LVT Viscometer at 25°C and at the indicated spindle speed and are reported in Table 5 (2.7 wt% polyacrylate a.m.) and Table 6 (2.25 wt% polyacrylate a.m.) .

The thickened liquid aqueous detergent compositions containing PAC3, PAC4 and PAC5 are uncoloured and crystal clear, while the thickened liquid aqueous detergent composition containing PAC6 has light blue reflexes.

**Table 5 - BV (mPa$_*$s) and BYV (2.7 wt% a.m.)**

| | BV | | | |
|---|---|---|---|---|
| rpm | PAC3 | PAC4 | PAC5 | PAC6* |
| 0,5 | 42600 | 58400 | 512000 | 32000 |
| 1 | 30000 | 44400 | 288000 | 21600 |
| 5 | 15000 | 19120 | 108000 | 7750 |
| 10 | 8080 | 14600 | 71300 | 5700 |
| 20 | 5740 | 10750 | 44600 | 3750 |
| 50 | 3880 | 7260 | 25280 | 2330 |
| 100 | 2960 | 5520 | 16320 | 1638 |
| | BYV | | | |
| | 126 | 140 | 2240 | 104 |
| *comparative | | | | |

**Table 6 - BV (mPa$_*$s) and BYV (2.25 wt% a.m.)**

| | BV | | | |
|---|---|---|---|---|
| rpm | PAC3 | PAC4 | PAC5 | PAC6* |
| 0,5 | 20320 | 31200 | 62800 | 18000 |
| 1 | 13280 | 18500 | 49000 | 12000 |
| 5 | 6040 | 6950 | 31800 | 4000 |
| 10 | 4280 | 4760 | 22800 | 2600 |
| 20 | 3120 | 3340 | 13400 | 1810 |
| 50 | 2184 | 2240 | 7140 | 1090 |
| 100 | 1710 | 1650 | 4600 | 800 |
| | BYV | | | |
| | 70 | 127 | 138 | 60 |
| * comparative | | | | |

It should be noted that the shear rate, as indicated herein by spindle speed (rpm), is an important parameter in viscosity measurement and that all the compositions according to the disclosure are noticeably pseudoplastic, or shear thinning.

This behaviour and the associated BYVs reflect the suspending properties of the composition, which are sensibly improved.

### Example 8

The thickened liquid aqueous detergent compositions of Example 8 were prepared according to the following procedure.

The crosslinked alkali swellable polyacrylate (PAC4,) was prepared by emulsion polymerization and dispersed in deionized water at 1.5 wt% a.m.. The detergent component is added (10.7 wt% a.m. of Sodium Laureth Sulfate and 2.4 wt% a.m. of Cocoamidopropyl Betaine) and mixed, then the pH is adjusted to 6.6-6.8 by addition of NaOH.

The amount of electrolyte (NaCl) reported in Table 7 (wt%) is added.

The solution was mixed by stirring for about 30 minutes and allowed to stand overnight before the viscosity is measured.

Five compositions with different amount of NaCl were tested.

The viscosities (BV, mPa∗s) of the thus obtained thickened liquid aqueous detergent compositions were measured using a Brookfield(R) Model LVT Viscometer at 25°C and at the indicated spindle speed and are reported in Table 7. All compositions were perfectly colorless and clear.

#### Table 7 - BV (mPa∗s) (1.5 wt% a.m.)

| rpm | \multicolumn{5}{c}{BV} |
| --- | --- | --- | --- | --- | --- |
| | 0% NaCl | 0.5% NaCl | 1.0% NaCl | 1.5% NaCl | 2.0% NaCl |
| 0,5 | 6000 | 6800 | 14880 | 26640 | 44400 |
| 1 | 4900 | 5120 | 11840 | 21200 | 34400 |
| 5 | 2500 | 2920 | 7024 | 13600 | 22240 |
| 10 | 1900 | 2280 | 5780 | 11720 | 18520 |
| 20 | 1625 | 2000 | 4865 | 10500 | 15760 |
| 50 | 1100 | 1472 | 4184 | 8740 | 13260 |
| 100 | 900 | 1382 | 3600 | 7500 | 12000 |

### Claims

1. Liquid aqueous detergent compositions comprising:

    i) from 0.2 to 10 % by weight of a thickening agent which is a crosslinked alkali swellable polyacrylate obtainable by polymerization of:

        a) from 20 to 70% by weight of a monoethylenically unsaturated monomer containing a carboxylic group;
        b) from 20 to 70% by weight of a (meth)acrylic acid ester;
        c) from 0.05 to 3% by weight of an unsaturated monomer containing one or more acetoacetyl or cyanoacetyl groups;
        d) from 0.01 to 3% by weight of a polyethylenically unsaturated monomer;
        e) from 0 to 10% by weight of a nonionic acrylic associative monomer;

    ii) from 5 to 60% by weight of a detergent component consisting of at least one compound selected from anionic surfactants, amphoteric surfactants, cationic surfactants, zwitterionic surfactants, non-ionic surfactants and mixture thereof.

2. Liquid aqueous detergent compositions according to claim 1 comprising:

i) from 0.5 to 8 % by weight of a thickening agent which is a crosslinked alkali swellable polyacrylate obtainable by polymerization of:

**a)** from 20 to 50 % by weight of a monoethylenically unsaturated monomer containing a carboxylic group;
**b)** from 40 to 70 % by weight of a (meth)acrylic acid ester;
**c)** from 0.1 to 2 % by weight of an unsaturated monomer containing one or more acetoacetyl or cyanoacetyl groups;
**d)** from 0.02 to 1 % by weight of a polyethylenically unsaturated monomer;
**e)** from 0 to 10% by weight of a nonionic acrylic associative monomer;

ii) from 5 to 20 % by weight of the detergent component.

3. Liquid aqueous detergent compositions according to claim 1 in which the unsaturated monomer containing one or more acetoacetyl or cyanoacetyl groups is an acetoacetoxyalkyl (meth)acrylate) of formula (I)

$$CH_2=CR\text{-}CO\text{-}O\text{-}R_1\text{-}O\text{-}CO\text{-}CH_2CO\text{-}CH_3 \qquad (I)$$

in which R is H or $CH_3$ and $R_1$ is a $C_2$-$C_4$ linear or branched alkylene radical group.

4. Liquid aqueous detergent compositions according to claim 3 in which in formula (I) R is $CH_3$ and $R_1$ is the ethylene radical.

5. Liquid aqueous detergent compositions according to claim 1 in which the monoethylenically unsaturated monomer containing a carboxylic group is acrylic acid, methacrylic acid, itaconic acid or mixture thereof and the (meth)acrylic acid ester is a $C_1$-$C_{40}$ (meth)acrylic acid alkyl ester.

6. Liquid aqueous detergent compositions according to claim 5 in which the monoethylenically unsaturated monomer containing a carboxylic group is methacrylic acid and the (meth)acrylic acid ester is ethyl acrylate.

7. Liquid aqueous detergent compositions according to claim 1 in which the crosslinked alkali swellable polyacrylate is obtainable by polymerization of:

**a)** from 20 to 70% by weight of a monoethylenically unsaturated monomer containing a carboxylic group;
**b)** from 20 to 70% by weight of a (meth)acrylic acid ester;
**c)** from 0.1 to 0.5% by weight of an unsaturated monomer containing one or more acetoacetyl or cyanoacetyl groups;
**d)** from 0.01 to 3% by weight of a polyethylenically unsaturated monomer;
**e)** from 1.0 to 2.0% by weight of a nonionic acrylic associative monomer.

8. Use of a crosslinked alkali swellable polyacrylate obtainable by polymerization of:

**a)** from 20 to 70% by weight of a monoethylenically unsaturated monomer containing a carboxylic group;
**b)** from 20 to 70% by weight of a (meth)acrylic acid ester;
**c)** from 0.05 to 3% by weight of an unsaturated monomer containing one or more acetoacetyl or cyanoacetyl groups;
**d)** from 0.01 to 3% by weight of a polyethylenically unsaturated monomer;
**e)** from 0 to 10% by weight of a nonionic acrylic associative monomer, for thickening a liquid aqueous detergent composition comprising from 5 to 60% by weight of a detergent component consisting of at least one compound selected from anionic surfactants, amphoteric surfactants, cationic surfactants, zwitterionic surfactants and non-ionic surfactants and mixture thereof.

9. Use according to claim 8 of a crosslinked alkali swellable polyacrylate obtainable by polymerization of:

**a)** from 20 to 50 % by weight of a monoethylenically unsaturated monomer containing a carboxylic group;
**b)** from 40 to 70 % by weight of a (meth)acrylic acid ester;
**c)** from 0.1 to 2 % by weight of an unsaturated monomer containing one or more acetoacetyl or cyanoacetyl groups;
**d)** from 0.02 to 1 % by weight of a polyethylenically unsaturated monomer;

**e)** from 0 to 10% by weight of a nonionic acrylic associative monomer,
for thickening a liquid aqueous detergent composition comprising from 5 to 20 % by weight of the detergent component.

**10.** Use according to claim 8 in which the unsaturated monomers containing one or more acetoacetyl or cyanoacetyl groups is an acetoacetoxyalkyl (meth)acrylate) of formula (I)

$$CH_2=CR\text{-}CO\text{-}O\text{-}R_1\text{-}O\text{-}CO\text{-}CH_2CO\text{-}CH_3 \qquad (I)$$

in which R is H or $CH_3$ and $R_1$ is a $C_2C_4$ linear or branched alkylene radical group.

**11.** Use according to claim 10 in which in formula (I) R is $CH_3$ and $R_1$ is the ethylene radical.

**12.** Use according to claim 8 in which the monoethylenically unsaturated monomer containing a carboxylic group is acrylic acid, methacrylic acid, itaconic acid or mixture thereof and the (meth)acrylic acid ester is a $C_1$-$C_{40}$ (meth)acrylic acid alkyl ester.

**13.** Use according to claim 12 in which the monoethylenically unsaturated monomer containing a carboxylic group is methacrylic acid and the (meth)acrylic acid ester is ethyl acrylate.

**14.** Use according to claim 8 in which the crosslinked alkali swellable polyacrylate is obtainable by polymerization of:

**a)** from 20 to 70% by weight of a monoethylenically unsaturated monomer containing a carboxylic group;
**b)** from 20 to 70% by weight of a (meth)acrylic acid ester;
**c)** from 0.1 to 0.5% by weight of an unsaturated monomer containing one or more acetoacetyl or cyanoacetyl groups;
**d)** from 0.01 to 3% by weight of a polyethylenically unsaturated monomer;
**e)** from 1.0 to 2.0% by weight of a nonionic acrylic associative monomer.

**15.** Use according to any of claims from 8 to 14 in which the liquid aqueous detergent composition is a personal care detergent.

**16.** Use according to any of claims from 8 to 14 in which the liquid aqueous detergent composition is a household detergent.

**17.** Use according to any of claims from 8 to 14 in which the liquid aqueous detergent composition is a heavy duty detergent.

**Patentansprüche**

**1.** Flüssige, wässrige Detergenziengemische, umfassend:

i) 0,2 bis 10% Gewichtsanteil eines Verdickungsmittels, bei dem es sich um ein vernetztes alkalisch quellendes Polyacrylat handelt, das erhalten werden kann durch Polymerisation von:

a) 20 bis 70 % Gewichtsanteil eines monoethylenisch ungesättigten Monomers, enthaltend eine Carboxygruppe;
b) 20 bis 70 % Gewichtsanteil eines Acrylsäure(methyl)esters;
c) 0,05 bis 3 % Gewichtsanteil eines ungesättigten Monomers, enthaltend eine oder mehrere Acetoacetyl- oder Cynoacetylgruppen;
d) 0,01 bis 3 % Gewichtsanteil eines polyethylenisch ungesättigten Monomers;
e) 0 bis 10 % Gewichtsanteil eines nicht ionischen assoziativen Acrylmonomers;

ii) 5 bis 60 % Gewichtsanteil einer Reinigungsmiltelkomponente, bestehend aus mindestens einer Verbindung, ausgewählt aus anionischen Tensiden, amphoterischen Tensiden, kationischen Tensiden, zwitterionischen Tensiden, nicht ionischen Tensiden und einer Mischung daraus.

**2.** Flüssige, wässrige Detergenziengemische nach Anspruch 1, umfassend:

i) 0,5 bis 8 % Gewichtsanteil eines Verdickungsmittels, bei dem es sich um ein vernetztes alkalisch quellendes Polyacrylat handelt, das erhalten werden kann durch Polymerisation von:

a) 20 bis 50 % Gewichtsanteil eines monoethylenisch ungesättigten Monomers, enthaltend eine Carboxygruppe;
b) 40 bis 70 % Gewichtsanteil eines Acrylsäure(methyl)esters;
c) 0,1 bis 2 % Gewichtsanteil eines ungesättigten Monomers, enthaltend eine oder mehrere Acetoacetyl- oder Cynoacetylgruppen,
d) 0,02 bis 1 % Gewichtsanteil eines polyethylenisch ungesättigten Monomers;
e) 0 bis 10 % Gewichtsanteil eines nicht ionischen assoziativen Acrylmonomers;

ii) 5 bis 20 % Gewichtsanteil einer Detergenzienkomponente.

**3.** Flüssige, wässrige Detergenziengemische nach Anspruch 1, bei denen das eine oder mehrere Acetocetyl- oder Cynoacetylgruppen enthaltende ungesättigte Monomer ein Acetoacetoxylalkali(meth)acrylat) mit der Formel (I)

$$CH_2=CR-CO-O-R_1-O-CO-CH_2-CO-CH_3 \qquad (I)$$

ist, wobei R H ist oder $CH_3$ und $R_1$ eine $C_2$-$C_4$ lineare oder verzweigte Alkylradikalgruppe ist.

**4.** Flüssige, wässrige Detergenziengemische nach Anspruch 3, wobei R in der Formel (I) $CH_3$ ist und $R_1$ das Ethylenradikal ist.

**5.** Flüssige, wässrige Detergenziengemische nach Anspruch 1, wobei das eine Carboxygruppe enthaltende monoethylenisch ungesättigte Monomer Acrylsäure, Methacrylsäure, Itaconsäure oder eine Mischung daraus ist und der (Meth)Acrylsäureester ein $C_1$-$C_{40}$-(Meth)Acrylsäurealkylester ist.

**6.** Flüssige, wässrige Detergenziengemische nach Anspruch 5, wobei das eine Carboxygruppe enthaltende monoethylenisch ungesättigte Monomer Methacrylsäure ist und der (Meth)Acrylsäureester Ethylacrylat ist.

**7.** Flüssige, wässrige Detergenziengemische nach Anspruch 1, wobei das vernetzte alkalisch quellbare Polyacrylat erhalten werden kann durch Polymerisation von:

a) 20 bis 70 % Gewichtsanteil eines monoethylenisch ungesättigten Monomers, enthaltend eine Carboxygruppe;
b) 20 bis 70 % Gewichtsanteil eines Acrylsäure(methyl)esters;
c) 0,1 bis 0,5 % Gewichtsanteil eines ungesättigten Monomers, enthaltend eine oder mehrere Acetoacetyl- oder Cynoacetylgruppen;
d) 0,01 bis 3 % Gewichtsanteil eines polyethylenisch ungesättigten Monomers;
e) 1,0 bis 2,0 % Gewichtsanteil eines nicht ionischen assoziativen Acrylmonomers;

**8.** Verwendung eines vernetzten alkalisch quellenden Polyacrylats, das erhalten werden kann durch Polymerisation von:

a) 20 bis 70 % Gewichtsanteil eines monoethylenisch ungesättigten Monomers, enthaltend eine Carboxygruppe;
b) 20 bis 70 % Gewichtsanteil eines Acrylsäure(methyl)esters;
c) 0,05 bis 3 % Gewichtsanteil eines ungesättigten Monomers, enthaltend eine oder mehrere Acetoacetyl- oder Cynoacetylgruppen;
d) 0,01 bis 3 % Gewichtsanteil eines polyethylenisch ungesättigten Monomers;
e) 0 bis 10 % Gewichtsanteil eines nicht ionischen assoziativen Acrylmonomers zum Verdicken eines flüssigen wässrigen Detergenziengemischs, umfassend 5 bis 60% Gewichtsanteil einer Detergenzienkomponente, bestehend aus mindestens einer Verbindung, ausgewählt aus anionischen Tensiden, amphoterischen Tensiden, kationischen Tensiden, zwitterionischen Tensiden, nicht ionischen Tensiden und einer Mischung daraus.

**9.** Verwendung nach Anspruch 8 eines vernetzten alkalisch quellenden Polyacrylats, das erhalten werden kann durch Polymerisation von:

a) 20 bis 50 % Gewichtsanteil eines monoethylenisch ungesättigten Monomers, enthaltend eine Carboxygruppe;
b) 40 bis 70 % Gewichtsanteil eines Acrylsäure(methyl)esters;
c) 0,1 bis 2 % Gewichtsanteil eines ungesättigten Monomers, enthaltend eine oder mehrere Acetoacetyl- oder Cynoacetylgruppen;
d) 0,02 bis 1 % Gewichtsanteil eines polyethylenisch ungesättigten Monomers;
e) 0 bis 10 % Gewichtsanteil eines nicht ionischen assoziativen Acrylmonomer zum Verdicken eines flüssigen wässrigen Detergenziengemischs, umfassend 5 bis 20 % Gewichtsanteil einer Detergenzienkomponente.

**10.** Verwendung nach Anspruch 8, wobei das eine oder mehrere Acetocetyl- oder Cynoacetylgruppen enthaltende ungesättigte Monomer ein Acetoacetoxylalkali(meth)acrylat) mit der Formel (I)

$$CH_2=CR-CO-O-R_1-O-CO-CH_2-CO-CH_3 \qquad (I)$$

ist, wobei R H ist oder $CH_3$ und $R_1$ eine $CH_2CH_4$ lineare oder verzweigte Alkylradikalgruppe ist.

**11.** Verwendung nach Anspruch 10, wobei R in der Formel (I) $CH_3$ ist und $R_1$ das Ethylenradikal ist.

**12.** Verwendung nach Anspruch 8, wobei das eine Carboxygruppe enthaltende monoethylenisch ungesättigte Monomer Acrylsäure, Methacrylsäure, Itaconsäure oder eine Mischung daraus ist und der (Mekh)Acrylsäureester ein $C_1$-$C_{40}$-(Meth)Acrylsäurealkylester ist.

**13.** Verwendung nach Anspruch 12, wobei das eine Carboxygruppe enthaltende monoethylenisch ungesättigte Monomer Methacrylsäure ist und der (Meth)Acrylsäureester Ethylacrylat ist.

**14.** Verwendung nach Anspruch 8, wobei das vernetzte alkalisch quellbare Polyacrylat erhalten werden kann durch Polymerisation von:

a) 20 bis 70 % Gewichtsantell eines monoethylenisch ungesättigten Monomers, enthaltend eine Carboxygruppe;
b) 20 bis 70 % Gewichtsanteil eines Acrylsäure(methyl)esters;
c) 0,1 bis 0,5% Gewichtsantell eines ungesättigten Monomers, enthaltend eine oder mehrere Acetoacetyl- oder Cynoacetylgruppen;
d) 0,01 bis 3 % Gewichtsanteil eines polyethylenisch ungesättigten Monomers;
e) 1,0 bis 2,0% Gewichtsanteil eines nicht ionischen assoziativen Acrylmonomers.

**15.** Verwendung nach einem der Ansprüche 8 bis 14, wobei das flüssige, wässrige Detergenziengemisch ein Reinigungsmittel zur persönlichen Pflege ist.

**16.** Verwendung nach einem der Ansprüche 8 bis 14, wobei das flüssige, wässrige Detergerrziengemisch ein Haushaltsreinigungsmittel ist.

**17.** Verwendung nach einem der Ansprüche 8 bis 14, wobei das flüssige, wässrige Detergenziengemisch ein Industriereiniger ist.

**Revendications**

**1.** Compositions détergentes liquides aqueuses camprenank :

i) de 0,2 à 10 % en poids d'un agent épaississant étant un polyacrylate réticulé gonflable en milieu alcalin obtenu par polymérisation de :

a) 20 à 70 % en poids d'un monomère monoéthyléniquement insaturé contenant un groupe carboxylique ;
b) 20 à 70% en poids d'un ester d'acide (méth)acrylique ;
c) 0,05 à 3 % en poids d'un monomère insaturé contenant un ou plusieurs groupes acétoacétyles ou cyanoacétyles ;
d) 0,01 à 3 % en poids d'un monomère polyéthyléniquement insaturé ;
e) 0 à 10 % en poids d'un monomère associatif acrylique non ironique ;

ii) 5 à 60 % en poids d'un composant détergent constitué d'au moins un composé choisi parmi des tensioactifs anioniques, amphotères, cationiques, zwittérioniques, non ioniques ou un mélange de ceux-ci.

2. Compositions détergentes liquides aqueuses selon la revendication 1 comprenant :

i) de 0,5 à 8 % en poids d'un agent épaississant étant un polyacrylate réticulé gonflable en milieu alcalin obtenu par polymérisation de :

    a) 20 à 50 % en poids d'un monomère monoéthyléniquement insaturé contenant un groupe carboxylique ;
    b) 40 à 70 % en poids d'un ester d'acide (méth)acrylique ;
    c) 0,1 à 2 % en poids d'un monomère insaturé contenant un ou plusieurs groupes acétoacétyles ou cyanoacétyles ;
    d) 0,02 à 1 % en poids d'un monomère polyéthyléniquement insaturé ;
    e) 0 à 10 % en poids d'un monomère associatif acrylique non ioniques ;

ii) 5 à 20 % en poids du composant détergent.

3. Compositions détergentes liquides aqueuses selon la revendication 1, dans lesquelles le monomère insaturé contenant un ou plusieurs groupes acétoacétyles ou cyanoacétyles est un (méth)acrylate acétoacétoxyalkyles de formule (I)

$$CH_2=CR\text{-}CO\text{-}O\text{-}R_1\text{-}O\text{-}CO\text{-}CH_2\text{-}CO\text{-}CH_3 \qquad (I)$$

où R est H ou $CH_3$ et $R_1$ est un groupe radical alkyle ramifié ou linéaire en $C_2$-$C_4$.

4. Compositions détergentes liquides aqueuses selon la revendication 3, dans laquelle dans la formule (I) R est $CH_3$ et $R_1$ est le radical éthylène.

5. Compositions détergentes liquides aqueuses selon la revendication 1, dans lesquelles le monomère monoéthyléniquement insaturé contenant un groupe carboxylique est un acide acrylique, un acide (méth)acrylique, itaconique ou un mélange de ceux-ci et l'acide d'acide méth(acrylique) est un ester alkylique d'acide (méth)acrylique en $C_1C_{40}$,

6. Compositions détergentes liquides aqueuses selon la revendication 5, dans lesquelles le monomère monoéthyléniquement insaturé contenant un groupe carboxylique est un acide méthacrylique et l'ester d'acide (méth)acrylique est l'acrylate éthyle.

7. Compositions détergentes liquides aqueuses selon la revendication 1, dans lequel le polyacrylate réticulé gonflable en milieu alcalin peut être obtenu par polymérisation de :

    **a)** 20 à 70 % en poids d'un monomère monoéthyléniquement insaturé contenant un groupe carboxylique ;
    **b)** 20 à 70% en poids d'un ester d'acide (méth)acrylique ;
    **c)** 0,1 à 0,5 % en poids d'un monomère insaturé contenant un ou plusieurs groupes acétoacétyles ou cyanoacétyles ;
    **d)** 0,01 à 3 % en poids d'un monomère polyéthyléniquement insaturé ;
    **e)** 1,0 à 2,0 % en poids d'un monomère associatif acrylique non ionique.

8. Utilisation d'un polyacrylate réticulé gonflable en milieu alcalin pouvant être obtenu par polymérisation de :

    a) 20 à 70 % en poids d'un monomère monoéthyléniquement insaturé contenant un groupe carboxylique ;
    b) 20 à 70% en poids d'un ester d'acide (méth)acrylique ;
    c) 0,05 à 3 % en poids d'un monomère insaturé contenant un ou plusieurs groupes acétoacétyles ou cyanoacétyles ;
    d) 0,01 à 3% en poids d'un monomère polyéthyléniquement insaturé ;
    e) 0 à 10 % en poids d'un monomère associatif acrylique non ionique, pour l'épaississement d'une composition détergente liquide aqueuse comprenant de 5 à 60 % en poids d'un composant détergent constitué d'au moins un composé choisi parmi des tensioactifs anioniques, amphotères, cationiques, zwittérioniques et non ioniques ou un mélange de ceux-ci.

9. Utilisation selon la revendication 8 d'un polyacrylate réticulé gonflable en milieu alcalin pouvant être obtenu par polymérisation de :

   a) 20 à 50 % en poids d'un monomère monoéthyléniquement insaturé contenant un groupe carboxylique ;
   b) 40 à 70 % en poids d'un ester d'acide (méth)acrylique ;
   c) 0,1 à 2 % en poids d'un monomère insaturé contenant un ou plusieurs groupes acétoacétyles ou cyanoacétyles ;
   d) 0,02 à 1 % en poids d'un monomère polyéthyléniquement insaturé ;
   e) 0 à 10 % en poids d'un monomère associatif acrylique non ionique, pour l'épaississement d'une composition détergente liquide aqueuse comprenant de 5 à 20 % en poids du composant détergent.

10. Utilisation selon la revendication 8, dans laquelle les monomères insaturés contenant un ou plusieurs groupes acétoacétyles ou cyanoacétyles sont un (méth)acrylate acétoacétoxyalkyles de formule (I)

$$CH_2=CR\text{-}CO\text{-}O\text{-}R_1\text{-}O\text{-}CO\text{-}CH_2\text{-}CO\text{-}CH_3 \qquad (I)$$

où R est H ou $CH_3$ et $R_1$ est un groupe radical alkyle ramifié ou linéaire en $C_2$-$C_4$.

11. Utilisation selon la revendication 10, dans laquelle dans la formule (I) R est $CH_3$ et $R_1$ est le radical éthylène.

12. Utilisation selon la revendication 8, dans laquelle le monomère monoéthyléniquement insaturé contenant un groupe carboxylique est un acide acrylique, un acide (méth)acrylique, itaconique ou un mélange de ceux-ci et l'ester d'acide méth(acrylique) est un ester alkylique d'acide (méth)acrylique en $C_1$-$C_{40}$.

13. Utilisation selon la revendication 12, dans laquelle le monomère monoéthyléniquement insaturé contenant un groupe carboxylique est un acide méthacrylique et l'ester d'acide (méth)acrylique est l'acrylate éthyle.

14. Utilisation selon la revendication 8, dans laquelle le polyacrylate réticulé gonflable en milieu alcalin peut être obtenu par polymérisation de :

   **a)** 20 à 70 % en poids d'un monomère monoéthyléniquement insaturé contenant un groupe carboxylique ;
   **b)** 20 à 70% en poids d'un ester d'acide (méth)acrylique ;
   **c)** 0,1 à 0,5% en poids d'un monomère insaturé contenant un ou plusieurs groupes acétoacétyles ou cyanoacétyles ;
   **d)** 0,01 à 3 % en poids d'un monomère polyéthyléniquement insaturé ;
   **e)** 1,0 à 2,0 % en poids d'un monomère associatif acrylique non ionique.

15. Utilisation selon l'une quelconque des revendications de 8 à 14, dans lesquelles la composition détergente liquide aqueuse est un détergent de soins personnels.

16. Utilisation selon l'une quelconque des revendications de 8 à 14, dans lesquelles la composition détergente liquide aqueuse est un détergent ménager.

17. Utilisation selon l'une quelconque des revendications de 8 à 14, dans lesquelles la composition détergente liquide aqueuse est un détergent universel puissant.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 870994 A
- EP 13836 A
- US 4529773 A
- US 4138381 A
- US 6140438 A
- EP 1272159 A
- US 5516453 A
- US 4325856 A
- US 4654397 A
- US 4814373 A